**Europäisches Patentamt**

**European Patent Office**

**Office européen des brevets**

(19)

(11) Veröffentlichungsnummer: **0 114 267**
**B1**

(12) # EUROPÄISCHE PATENTSCHRIFT

(45) Veröffentlichungstag der Patentschrift:
**25.06.86**

(51) Int. Cl.⁴: **C 12 Q 1/32,** C 12 Q 1/00,
C 12 Q 1/28

(21) Anmeldenummer: **83112171.0**

(22) Anmeldetag: **03.12.83**

(54) **Testsystem und Verfahren zur Bestimmung von NAD(P)H.**

(30) Priorität: **24.12.82 DE 3247894**

(43) Veröffentlichungstag der Anmeldung:
**01.08.84 Patentblatt 84/31**

(45) Bekanntmachung des Hinweises auf die Patenterteilung:
**25.06.86 Patentblatt 86/26**

(84) Benannte Vertragsstaaten:
**BE CH DE FR GB IT LI NL SE**

(56) Entgegenhaltungen:
**EP - A - 0 008 342**
**EP - A - 0 090 223**
**DE - A - 2 940 165**

(73) Patentinhaber: **Merck Patent Gesellschaft mit beschränkter Haftung, Frankfurter Strasse 250, D-6100 Darmstadt (DE)**

(72) Erfinder: **Limbach, Berthold, Dr., Otto-Hahn-Strasse 8, D-6104 Seeheim 3 (DE)**
Erfinder: **Helger, Roland, Dr., Ludwighöhstrasse 85, D-6100 Darmstadt (DE)**

**Beschreibung**

Die Erfindung betrifft ein Testsystem und ein Verfahren mit erweitertem Messbereich zur Bestimmung von NAD(P)H bzw. von unter Bildung oder Verbrauch von NAD(P)H reagierenden Substraten oder Enzymen.

Die Bestimmung klinischer Parameter erfolgt in der Mehrzahl der Fälle über hochspezifische Dehydrogenase-Reaktionen, in deren Verlauf mittel- oder unmittelbar NAD(P)H gebildet oder verbraucht wird. Die dabei umgesetzte Menge NAD(P)H, absolut oder pro Zeiteinheit, ist ein Mass für die Konzentration der zu untersuchenden Substanz in einer Flüssigkeit. Als besonders vorteilhaft erweist sich die Dehydrogenase-Reaktion in Bezug auf Stöchiometrie und geringe Störanfälligkeit. Nachteilig ist dagegen, dass aufgrund der Absorptionseigenschaften des Coenzymmoleküls eine Auswertung der Messreaktion nur mit Photometern mit UV-Messbereich möglich ist; eine rein visuelle Auswertung kann nicht erfolgen. Dies wird erst möglich durch Kopplung der eigentlichen NAD(P)H-bildenden Reaktion mit einer Farbreaktion. Es sind eine Vielzahl von Verfahren beschrieben, die dies durch direkte oder mit Hilfe von Elektronenüberträgern wie Phenazinmethosulfat oder Diaphorase vermittelte Übertragung der Redoxäquivalente auf unterschiedliche Redoxindikatoren erreichen. Zu diesen Substanzen zählen z.B. Cytochrome, komplexierte oder chelatisierte Eisenionen, Dichlorphenolindophenol, Tetrazoliumsalze usw. Bekannt ist auch die Kopplung der Messreaktion mit einer Reaktionsfolge (DE-AS 15 98 263, EP-A 54 146), wobei die Farbstoffbildung über eine intermediär gebildete Substanz erfolgt. Allen diesen Systemen ist ein sequentieller Reaktionsverlauf gemeinsam, bei dem jeweils das Produkt der ersten Teilreaktion Ausgangssubstanz der zweiten usw. ist.

In der Patentanmeldung DE-A 32 11 167 (EP-A 90223) ist ein Verfahren beschrieben, bei dem eine zu bestimmende Substanz zu verschiedenen unterscheidbaren Produkten umgesetzt wird, so dass ein gegenüber den üblichen Tests grösserer Messbereich bei gleicher Messgenauigkeit erhalten wird. Dies wird durch Verwendung mehrerer, unabhängig voneinander die gleiche zu bestimmende Substanz umsetzender Enzymsysteme erreicht, wobei eines eine NAD-abhängige, ein anderes eine NAD-unabhängige Dehydrogenase enthält. Der einzige Nachteil dieses Verfahrens besteht darin, dass für einen grossen Teil der zu untersuchenden Substanzen in der klinischen Diagnostik die erforderlichen NAD-unabhängigen Enzyme — im Gegensatz zu den entsprechenden NAD-abhängigen Enzymen — nicht im Handel erhältlich sind.

Der Erfindung liegt die Aufgabe zugrunde, ein Testsystem zur Bestimmung von NAD(P)H zu entwickeln, das einen gegenüber den üblichen Systemen vielfach grösseren Messbereich hat, und das mit jederzeit im Handel erhältlichen Enzymen durchgeführt werden kann.

Überraschenderweise wurde gefunden, dass unter bestimmten Bedingungen eine unabhängige Umsetzung von NAD(P)H mit verschiedenen Redoxindikatoren möglich ist. Unabhängig bedeutet dabei, dass z.B. die Umsetzung des zweitens Redoxindikators (mit dem niedrigeren elektrochemischen Potential) erst nach weitgehend vollständiger Umsetzung des ersten (mit dem höheren elektrochemischen Potential) erfolgt, so dass eine getrennte Auswertung möglich ist. Dies war umso überraschender, als zu erwarten war, dass bei gleichzeitigem Vorliegen der Redoxindikatoren durch die Wechselwirkung der einzelnen Subtanzen untereinander und durch fremde Einflüsse (Sauerstoff, Testsubstanzen) Störungen auftreten.

Gegenstand der Erfindung ist somit ein Testsystem mit erweitertem Messbereich zur Bestimmumg von NAD(P)H bzw. von unter Bildung oder Verbrauch von NAD(P)H reagierende Substraten oder Enzymen, das dadurch gekennzeichnet ist, dass es gleichzeitig mehrere, unabhängig voneinander gegenüber NAD(P)H als Elektronenakzeptor fungierende Substanzen mit verschiedenen elektrochemischen Potentialen enthält.

Ein weiterer Gegenstand der Erfindung sind Verfahren zur Bestimmung von NAD(P)H bzw. von unter Bildung oder Verbrauch von NAD(P)H reagierenden Substraten oder Enzymen in wässriger Lösung, die dadurch gekennzeichnet sind, dass die Probelösung gleichzeitig mit mehreren, unabhängig voneinander gegenüber NAD(P)H als Elektronenakzeptor fungierenden Substanzen mit verschiedenen elektrochemischen Potentialen behandelt wird, wobei verschiedene analytisch unterscheidbare Endprodukte entstehen, die messtechnisch oder visuell ausgewertet werden.

Je nach Art des Redoxindikators kann die Übertragung der Elektronen direkt oder mit Hilfe eines sogenannten Elektronenüberträgers erfolgen. Vorzugsweise enthält das Testsystem Elektronenüberträger, die eine katalytische Wirkung auf die Redoxindikatoren ausüben. Geeignete Elektronenüberträger sind z.B. Phenazinmethosulfat (PMS), Phenazinethosulfat (PES), Methoxyphenazinmethosulfat (MPMS), Meldolablau, Diaphorase usw. sowie Gemische dieser Substanzen.

Als Redoxindikatoren kommen prinzipiell alle Substanzen in Frage, die ein höheres elektrochemisches Potential als NAD(P)H/NAD(P) besitzen und deren oxidierte und reduzierte Form visuell, photometrisch oder mit elektrochemischen Verfahren eindeutig zu unterscheiden sind. Geeignete Redoxindikatoren für das erfindungsgemässe Testsystem sind z.B. Oxazin- und Thiazinfarbstoffe, Tetrazoliumsalze usw. [DE-PS 28 34 743, Proc. Soc. Exp. Biol. *104*, 407 (1960)], vorzugsweise Dichlorphenolindophenol (DIP), 3-(4--Jodphenyl)-2-(4-nitrophenyl)-5-phenyl-2H-tetrazoliumchlorid (INT) und 3-(4,5-Dimethylthiazol-2-yl)--2,5-diphenyl-2H-tetrazoliumbromid (MTT). Die Verwendung dieser Redoxindikatoren in Dehydrogenase-Testsystemen war bisher auf Systeme mit nur einem Redoxindikator beschränkt. Überraschenderweise konnte nun gezeigt werden, dass bei Vorliegen mehrerer Redoxindikatoren mit verschiedenen Normalpotentialen Testsysteme mit erweitertem Messbereich zur Verfügung gestellt werden können. Die Normalpotentiale der Redoxindikatoren sollten zwischen —0,3 und +0,5, insbesondere zwischen —0,3 und +0,3 liegen. Die Bestimmung wird in der Regel in gepufferten wässrigen Lösungen durchgeführt, wobei der ph-Wert zwischen 4,5 und 8,0, vorzugsweise zwischen 5,0 und 7,0 liegen sollte. Geeignete Puffer zur Einstellung dieser pH-Werte sind z.B. Phosphatpuffer, Citratpuffer, 2-(N-Morpholino)-ethansulfonsäure-Natriumsalz, Bis-(2-hydroxyethyl)--amino-tris(hydroxymethyl)methan, Piperazin-N,N'--bis(2-ethansulfonsäure)-Dikaliumsalz usw., vorzugsweise Phosphatpuffer und Citratpuffer.

Die Auswahl der für das erfindungsgemässe Testsystem verwendeten Redoxindikatoren erfolgt nach der Unterscheidbarkeit der bei der Umsetzung gebildeten Messsignale, vorzugsweise nach der Extinktionsänderung bei verschiedenen Wellenlängen (photometrische Auswertung) oder nach der unterscheidbaren Farbänderung der Testlösung (visuelle Auswertung). Im letzten Fall sind insbesondere Kombinationen von Redoxindikatoren geeignet, von denen je eine Form farblos bzw. nur schwach gefärbt ist. Nachstehend sind einige bevorzugte Redoxindikatoren mit ihren Normalpotentialen bei einem pH-Wert von 7 und die entsprechenden Farbänderungen aufgezählt.

| Substanz | Potential bei pH 7,0 | Farbe der oxidierten Form | Farbe der reduzierten Form |
|---|---|---|---|
| NADH | −0,32 | | |
| DIP | +0,23 | blau | farblos |
| MTT | +0,11 | farblos | blau |
| INT | +0,09 | farblos | rot |
| Thionin | +0,06 | violett | farblos |
| Methylenblau | +0,01 | blau | farblos |

Aus der Tabelle geht hervor, dass die Kombination der Redoxindikatoren DIP/INT mit einem Farbübergang in der 1. Stufe von blau nach farblos und in der 2. Stufe von farblos nach rot besonders vorteilhaft ist. Beide Substanzen besitzen sowohl zwischen oxidierter und reduzierter Form einer jeden Substanz als auch zwischen den Substanzen deutliche Unterschiede im Absorptionsspektrum und im visuellen Farbeindruck. Eine Umsetzung der beiden Substanzen im Gemisch mit NAD(P)H führt zu einer unabhängigen Umsetzung von DIP und INT und damit zu einem gegenüber einem System mit nur einer dieser Substanzen erheblich erweiterten Messbereich. Die Substanz mit dem höheren Normalpotential (DIP) wird nahezu vollständig vor der zweiten Substanz (INT) umgesetzt. Bei einer NAD(P)H-Bestimmung mit DIP als einzigem Redoxindikator wird ein Konzentrationsbereich bis etwa 0,26 mmol/l erfasst, mit INt ein Bereich bis etwa 0,13 mmol/l und mit der Kombination von DIP und INT ein Bereich bis etwa 0,55 mmol/l, d.h., dass mit dem erfindungsgemässen Testsystem ein doppelt bis mehr als 4facher Konzentrationsbereich von NAD(P)H erfasst wird. Die photometrische Auswertung kann z.B. über eine Registrierung der Extinktion bei zwei verschiedenen Wellenlängen (460 und 650 nm) erfolgen. Bei der visuellen Auswertung lässt sich eine weitere Steigerung der erhaltenen Farbstufen durch Zusatz eines sogenannten Hintergrundfarbstoffes (z.B. Titangelb) erhalten.

Bei der Kombination der Redoxindikatoren INT/Thionin erfolgt entsprechend den Normalpotentialen zuerst die Reduktion von INT und anschliessend diejenige von Thionin. Je nach dem gewählten Auswerteverfahren sind eine Vielzahl von Kombinationen von Redoxindikatoren für das erfindungsgemässe Verfahren verwendbar.

Das Testsystem nach der Erfindung kann prinzipiell für alle NAD(P)H-Bestimmungen sowie für alle NAD(P)H verbrauchenden oder bildenden Reaktionen verwendet werden. Die zu untersuchenden Proben sind vorzugsweise Körperflüssigkeiten wie Serum, Plasma, Urin usw. Die Durchführung des erfindungsgemässen Verfahrens erfolgt in der für enzymatische Methoden üblichen Weise, wobei man im allgemeinen zuerst die NAD(P)H-umsetzende Reaktion ablaufen lässt und danach das gebildete oder verbrauchte NAD(P)H bestimmt. Selbstverständlich ist bei NAD(P)H-bildenden Reaktionen auch eine direkte Kopplung möglich. Auch lassen sich Enzymaktivitäten dadurch bestimmen, dass die Reaktion zu einer bestimmten Zeit gestoppt und das in dieser Zeit gebildete oder verbrauchte NAD(P)H bestimmt wird. Die Redoxindikatoren werden vorzugsweise gleichzeitig zur Probelösung gegeben; eine zeitlich getrennte Zugabe ist ebenfalls möglich.

Das erfindungsgemässe Testsystem eignet sich auch in Form von damit imprägnierten saugfähigen Materialien als Indikator zur Bestimmung von NAD(P)H.

*Beispiel 1*

Bestimmung von NAD(P)H

Es werden die folgenden 3 Ansätze hergestellt:

Ansatz A   0,01    mmol/l PMS
          0,25    mmol/l DIP
          0,025   mmol/l Titangelb
          0,15    mol/l Citratpuffer pH 5,8

Ansatz B   0,01    mmol/l PMS
          0,2     mmol/l INT
          0,025   mmol/l Titangelb
          0,15    mol/l Citratpuffer pH 5,8

Ansatz C   0,01    mmol/l PMS
          0,2     mmol/l INT
          0,25    mmol/l DIP
          0,025   mmol/l Titangelb
          0,15    mol/l Citratpuffer pH 5,8

Zu je 2 ml dieser Ansätze werden verschiedene Mengen an NADH (NADPH) gegeben. Nach jeweils 10 Minuten werden die Extinktionen bei 460 und 650 nm sowie der Farbeindruck der jeweiligen Testlösung ermittelt. Abhängig von der eingesetzten NADH- bzw. NADH-Konzentration in der Gesamttestlösung ergeben sich für den Farbeindruck folgende Resultate:

| NADH (NADPH) [mmol/l] | Resultierender Farbeindruck der Testlösung | | |
|---|---|---|---|
| | Ansatz A | Ansatz B | Ansatz C |
| 0 | tiefblau | gelb | tiefblau |
| 0,05 | dunkelblau | orange | dunkelblau |
| 0,09 | blau | braun | blau |
| 0,13 | hellblau | rot | hellblau |
| 0,17 | blaugrün | | blaugrün |
| 0,22 | grün | | grün |
| 0,24 | gelbgrün | | gelbgrün |
| 0,26 | gelb | | ocker |
| 0,27 | | | orange |
| 0,30 | | | braun |
| 0,34 | | | rotbraun |
| 0,38 | | | rot |
| 0,55 | | | dunkelrot |

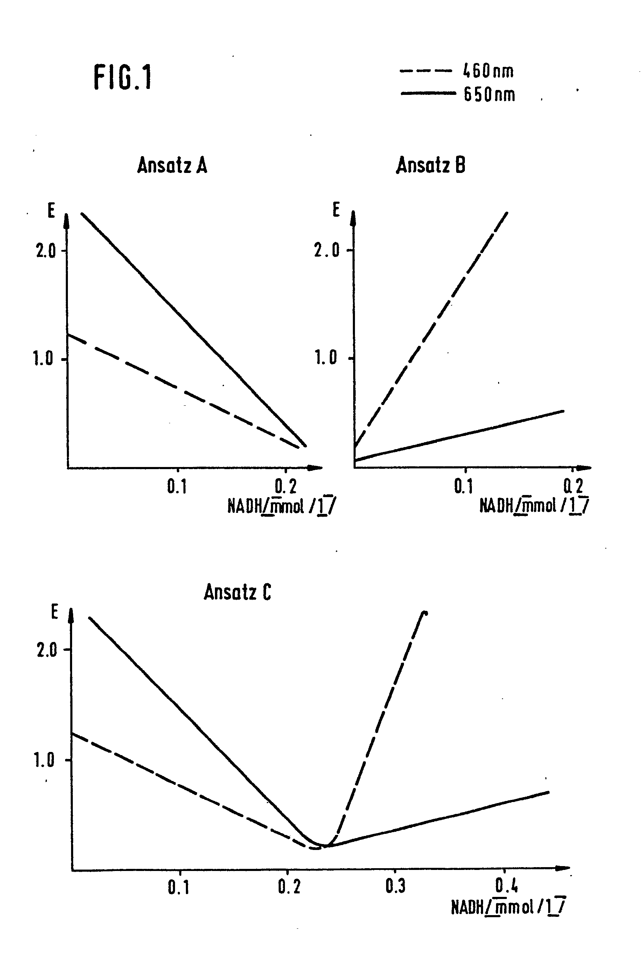

Die Änderung der Extinktion der jeweiligen Testlösung ist in Abb. 1 dargestellt.

Während mit den Ansätzen A und B eine visuelle Bestimmung der NAHD-Konzentration im Bereich bis maximal 0,25 mmol/l möglich ist, ist mit Ansatz C eine Bestimmung bis zu 0,55 mmol/l möglich.

*Beispiel 2*

Bestimmung von NADH

Zu 2 ml einer Lösung enthaltend

0,01  mmol/l  MPMS
0,2  mmol/l  INT
0,03  mmol/l  Thionin und
0,15  mol/l  Citratpuffer, pH 5,2

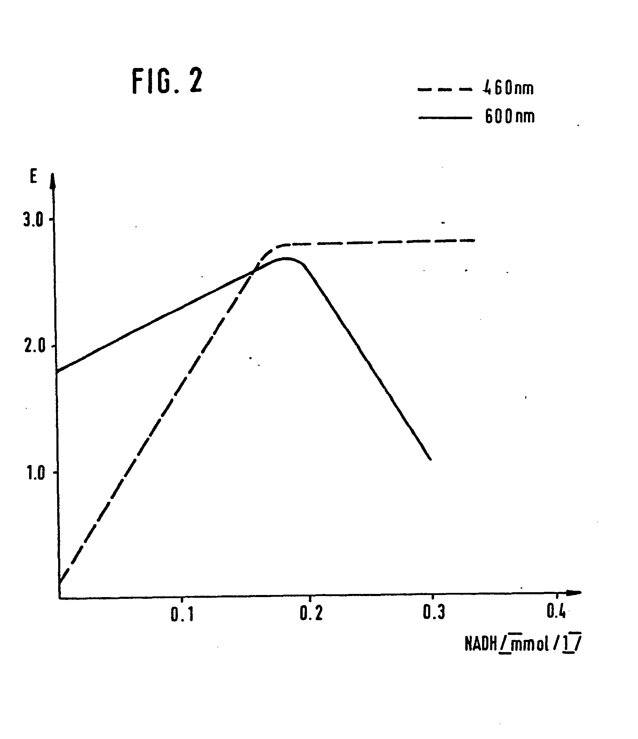

werden verschiedene Mengen NADH gegeben. Nach 10 Minuten werden die Extinktionen der jeweiligen Testlösungen bei 460 und 600 nm ermittelt. Die erhaltenen Resultate, abhängig von der eingesetzten NADH-Konzentration in der Gesamttestlösung, sind in Abb. 2 dargestellt.

Für den Testansatz mit beiden Farbstoffen erhält man einen etwa doppelt so grossen Messbereich für die NADH-Bestimmung als bei entsprechenden Testansätzen mit jeweils nur einem Farbstoff.

*Beispiel 3*

Bestimmung von NADH

Es werden die folgenden Ansätze hergestellt:

Ansatz A  0,01  mmol/l Meldolablau
          0,25  mmol/l DIP
          0,025 mmol/l Titangelb
          0,15  mol/l  Citratpuffer pH 5,8

Ansatz B  0,01  mmol/l Meldolablau
          0,25  mmol/l DIP
          0,2   mmol/l INT
          0,025 mmol/l Titangelb
          0,15  mol/l  Citratpuffer pH 5,8

Zu je 2 ml dieser Ansätze werden verschiedene Mengen an NADH gegeben. Nach jeweils 10 Minuten wird der Farbeindruck der jeweiligen Testlösung ermittelt. Abhängig von der eingesetzten NADH-Konzentration in der Gesamttestlösung ergeben sich für den Farbeindruck folgende Resultate:

| NADH [mmol/l] | Resultierender Farbeindruck der Testlösung | |
|---|---|---|
| | Ansatz A | Ansatz B |
| 0 | tiefblau | tiefblau |
| 0,10 | türkis | türkis |
| 0,20 | dunkelgrün | dunkelgrün |
| 0,25 | hellgrün | hellgrün |
| 0,30 | | hellbraun |
| 0,35 | | hellrot-violett |
| 0,40 | | weinrot |
| 0,50 | | dunkelrot |

Während mit Ansatz A eine visuelle Bestimmung der NADH-Konzentration im Bereich bis 0,25 mmol/l möglich ist, ermöglicht Ansatz B eine Bestimmung bis 0,5 mmol/l.

*Beispiel 4*

Bestimmung von Harnstoff im Serum

Zu 1,5 ml Reaktionslösung enthaltend

0,03   mol/l  Phosphatpuffer, pH 7,6
2,2   mmol/l  ADP
4,0   mmol/l  Ketoglutarat
0,5   mmol/l  NADH
30 KU/l Glutamatdehydrogenase und
30 KU/l Urease

werden 25 μl einer Serumprobe mit jeweils verschiedenem Harnstoffgehalt (eingestellt durch Zugabe von Harnstoff, überprüft mit Standardmethode) gegeben. Nach 10 Minuten werden jeweils 350 μl einer Lösung enthaltend

0,35   mol/l  Citratpuffer, pH 5,8
0,05  mmol/l  PMS
1,34  mmol/l  DIP
1,1   mmol/l  INT
0,13  mmol/l  Titangelb
250    U/l  Diaphorase

zugegeben und nach weiteren 5 bis 10 Minuten der Farbeindruck der Reaktionslösung im Durchlicht ermittelt. Man erhält folgende Resultate:

| Harnstoff [mg/dl] | Resultierender Farbeindruck der Testlösung |
|---|---|
| 0 | rot |
| 30 | rotbraun |
| 45 | braun |
| 55 | orange |
| 60 | ocker |
| 70 | gelbgrün |
| 80 | grün |
| 100 | blaugrün |
| 120 | hellblau |
| 150 | dunkelblau |
| 180 | tiefblau |

Während bei einem Testansatz mit nur einem Farbstoff visuelle Harnstoffbestimmungen bis zu 70 mg/dl durchgeführt werden können, erlaubt das erfindungsgemässe System Bestimmungen bis zu 180 mg/dl.

*Beispiel 5*

Bestimmung von Harnsäure im Urin

Zu 1,3 ml Reaktionslösung enthaltend

| | | |
|---|---|---|
| 0,03 | mol/l | Phosphatpuffer, pH 8,5 |
| 42 | mmol/l | KCl |
| 1,43 | mol/l | Ethanol |
| 0,5 | mmol/l | NADP$^+$ |
| 1750 | U/l | Katalase |
| 150 | U/l | Aldehyddehydrogenase und |
| 50 | U/l | Uricase |

werden 200 µl Urinproben mit verschiedenem Harnsäuregehalt gegeben (eingestellt durch Zugabe von Harnsäure, überprüft mit Standardverfahren). Nach 20 Minuten werden zu der jeweiligen Testlösung 350 µl einer Lösung gegeben, die folgende Bestandteile enthält:

| | | |
|---|---|---|
| 0,5 | mol/l | Citratpuffer, pH 5,8 |
| 0,05 | mmol/l | PES |
| 1,34 | mmol/l | DIP |
| 1,10 | mmol/l | INT und |
| 0,13 | mmol/l | Titangelb |

Nach weiteren 10 Minuten wird der Farbeindruck der Reaktionslösung im Durchlicht ermittelt. Man erhält folgende Resultate:

| Harnsäure [mg/dl] | Resultierender Farbeindruck der Testlösung |
|---|---|
| 0 | tiefblau |
| 9 | dunkelblau |
| 15 | blau |
| 21 | hellblau |
| 29 | blaugrün |
| 37 | grün |
| 40 | gelbgrün |
| 42 | ocker |
| 45 | braun |
| 50 | rotbraun |
| 57 | rot |
| 64 | dunkelrot |

Während bei einem entsprechenden Test mit nur einem Farbstoff visuelle Harnsäurebestimmungen in einem Bereich bis etwa 40 mg/dl durchgeführt werden können, sind mit dem erfindungsgemässen System bis über 60 mmol/l noch sicher erfassbar.

*Beispiel 6*

Bestimmung von Lactatdehydrogenase im Serum

Zu 1,2 ml Reaktionslösung enthaltend

| | | |
|---|---|---|
| 0,03 | mol/l | Phosphatpuffer, pH 7,5 |
| 0,6 | mmol/l | Pyruvat und |
| 0,5 | mmol/l | NADH |

werden 100 µl einer Serumprobe mit verschiedener LDH-Aktivität zugegeben (eingestellt durch Zugabe von LDH, überprüft mit Standardmethode). Nach 10 Minuten bei 25]C werden 300 µl einer Lösung zugegeben, die

| | | |
|---|---|---|
| 0,5 | mol/l | Citratpuffer, pH 5,8 |
| 1,34 | mmol/l | DIP |
| 1,10 | mmol/l | INT |
| 0,05 | mmol/l | PMS |
| 0,13 | mmol/l | Titangelb und |
| 4 | mmol/l | Oxamidsäure |

enthält. Nach weiteren 10 Minuten wird der Farbeindruck der jeweiligen Gesamttestlösung bestimmt. Man erhält folgende Resultate:

| LDH [U/l] | Resultierender Farbeindruck der Testlösung |
|---|---|
| 32 | rot |
| 96 | rotbraun |
| 160 | braun |
| 210 | orange |
| 225 | ocker |
| 255 | gelbgrün |
| 290 | grün |
| 370 | blaugrün |
| 430 | hellblau |
| 500 | blau |
| 560 | dunkelblau |
| 640 | tiefblau |

Während bei einem entsprechenden Test mit nur einem Farbstoff LDH-Bestimmungen mit visueller Auswertung in einem Bereich bis maximal 225 U/l durchgeführt werden können, sind mit dem erfindungsgemässen Testsystem Bestimmungen bis über 600 U/l möglich.

**Patentansprüche**

1. Testsystem mit erweitertem Messbereich zur Bestimmung von NAD(P)H, bzw. von unter Bildung oder Verbrauch von NAD(P)H reagierenden Substraten oder Enzymen, dadurch gekennzeichnet, dass es gleichzeitig mehrere, unabhängig voneinander gegenüber NAD(P)H als Elektronenakzeptor fungierende Substanzen mit verschiedenen elektrochemischen Potentialen enthält.

2. Testsystem nach Anspruch 1, dadurch gekennzeichnet, dass die als Elektronenakzeptoren fungierenden Substanzen Redoxindikatoren mit einem Normalpotential zwischen —0,3 und +0,5, vorzugsweise zwischen —0,3 und +0,3, sind.

3. Testsystem nach den Ansprüchen 1 und 2, dadurch gekennzeichnet, dass es zusätzlich einen Elektronenüberträger enthält.

4. Testsystem nach den Ansprüchen 1 bis 3, dadurch gekennzeichnet, dass es als Redoxindikatoren Dichlorphenolindophenol und ein Tetrazoliumsalz enthält.

5. Testsystem nach den Ansprüchen 1 bis 4, dadurch gekennzeichnet, dass es als Elektronenüberträger Phenazinmethosulfat, Phenazinethosulfat, Methoxyphenazinmethosulfat, Meldolablau, Diaphorase oder Gemische dieser Substanzen enthält.

6. Testsystem nach den Ansprüchen 1 bis 5, dadurch gekennzeichnet, dass es zusätzlich einen Hintergrundfarbstoff enthält.

7. Verfahren zur Bestimmung von NAD(P)H bzw. von unter Bildung oder Verbrauch von NAD(P)H reagierenden Substraten oder Enzymen in wässriger Lösung, dadurch gekennzeichnet, dass die Probelösung gleichzeitig mit mehreren, unabhängig voneinander gegenüber NAD(P)H als Elektronenakzeptoren fungierenden Substanzen mit verschiedenen elektrochemischen Potentialen behandelt wird, wobei verschiedene analytisch unterscheidbare Endprodukte entstehen, die messtechnisch oder visuell ausgewertet werden.

8. Verfahren nach Anspruch 7, dadurch gekennzeichnet, dass die Bestimmung in einer gepufferten wässrigen Lösung vom pH-Bereich 4,5-8, vorzugsweise 5,0-7, durchgeführt wird.

## Claims

1. Test system having an extended range of measurement for the determination of NAD(P)H, or of substrates or enzymes which react with the formation or consumption of NAD(P)H, characterised in that it contains, at one and the same time, several substances which, independently of one another, act as electron acceptors with respect to NAD(P)H and which have different electrochemical potentials.

2. Test system according to Claim 1, characterised in that the substances acting as electron acceptors are redox indicators having a standard potential between —0.3 and +0.5, preferably between —0.3 and +0.3.

3. Test system according to Claims 1 and 2, characterised in that it additionally contains an electron transfer agent.

4. Test system according to Claims 1 to 3, characterised in that it contains, as redox indicators, dichlorophenol-indophenol and a tetrazolium salt.

5. Test system according to Claims 1 to 4, characretised in that it contains, as electron transfer agents, phenazine methosulphate, phenazine ethosulphate, methoxyphenazine methosulphate, Meldola's blue, diaphorase or mixtures of these substances.

6. Test system according to Claims 1 to 5, characterised in that it additionally contains a background dyestuff.

7. Procedure for the determination of NAD(P)H, or of substrates or enzymes which react with the formation or consumption of NAD(P)H, in aqueous solution, characterised in that the sample solution is treated, at one and the same time, with several substances which act, independently of one another, as electron acceptors with respect to NAD(P)H and which have different electrochemical potentials, this giving rise to different end products which can be analytically differentiated and which are evaluated by a technique of measurement or visually.

8. Procedure according to Claim 7, characterised in that the determination is carried out in a buffered aqueous solution of pH range 4.5-8, preferably 5.0-7.

## Revendications

1. Système d'essai ayant un champ de mesure élargi pour la détermination de NAD(P)H ou d'enzymes ou de substrates réagissant avec formation ou consommation de NAD(P)H, caracrérisé en ce qu'il contient simultanément plusieurs subtances ayant des potentiels électrochimiques différents et agissant indépendamment l'une de l'autre vis-à-vis de NAD(P)H comme accepteur d'électrons.

2. Système d'essai selon la revendication 1, caractérisé en ce que les substances faisant office d'accepteurs d'électrons sont des indicateurs redox ayant un potentiel normal se situant entre —0,3 et +0,5, de préférence, entre —0,3 et +0,3.

3. Système d'essai selon les revendications 1 et 2, caractérisé en ce qu'il contient, en outre, un véhiculeur d'électrons.

4. Système d'essai selon les revendications 1 à 3, caractérisé en ce que, comme indicateurs redox, il contient du dichlorophénolindophénol et un sel de tétrazolium.

5. Système d'essai selon les revendications 1 à 4, caractérisé en ce que, comme véhiculeurs d'électrons, il contient du métosulfate de phénazine, de l'éthosulfate de phénazine, du méthosulfate de méthoxyphénazine, du bleu Meldola, de la diaphorase ou des mélanges de ces substances.

6. Système d'essai selon les revendications 1 à 5, caractérisé en ce qu'il contient, en outre un colorant de fond.

7. Procédé de détermination de NAD(P)H ou d'enzymes ou de substrats en solution aqueuse réagissant avec formation ou consommation de NAD(P)H, caractérisé en ce qu'on traite la solution d'échantillon simultanément avec plusieurs substances ayant des potentiels électrochimiques différents et agissant indépendamment l'une de l'autre vis-à-vis de NAD(P)H comme accepteur d'électrons, tandis qu'il se forme différents produits finals analytiquement différenciables que l'on évalue par la technique de mesure ou visuellement.

8. Procédé selon la revendication 7, caractérisé en ce que la détermination est effectuée dans une solution aqueuse tamponnée d'un pH se situant dans le domaine de 4,5-8, de préférence, de 5,0-7.

# FIG.1

- - - - 460nm
——— 650nm

## Ansatz A

## Ansatz B

## Ansatz C

# FIG. 2

– – – 460 nm
——— 600 nm

NADH /̄mmol / 1̄/